# EUROPÄISCHE PATENTSCHRIFT

(11) **EP 1 057 461 B1**
(45) Veröffentlichungstag und Bekanntmachung des Hinweises auf die Patenterteilung: **30.11.2005**
(21) Anmeldenummer: 00109206.3
(22) Anmeldetag: 28.04.2000
(51) Int. Cl.: A61F 2/30

(54) **Hüftgelenkprothese**
HIP JOINT PROSTHESIS
PROTHESE DE LA HANCHE

(30) Priorität: 29.05.1999 DE 19924676
(43) Veröffentlichungstag der Anmeldung: 06.12.2000
(73) Patentinhaber: Wolter, Dietmar, Prof. Dr., D-21033 Hamburg (DE)
(72) Erfinder: Wolter, Dietmar, Prof. Dr., D-21033 Hamburg (DE)
(74) Vertreter: Hauck, Graalfs, Wehnert, Döring, Siemons

(56) Entgegenhaltungen:
- DE-A- 3 741 490
- DE-U- 29 502 961
- GB-A- 2 315 222
- US-A- 4 731 088
- US-A- 5 389 107
- US-A- 5 514 182
- US-A- 5 702 474
- US-A- 5 755 807

## Beschreibung

Die Erfindung betrifft eine Endoprothese für das menschliche Hüftgelenk.

Künstliche Hüftgelenke werden in der Chirurgie und Orthopädie dann verwendet, wenn das eigentliche Hüftgelenk durch Erkrankungen, Verschleiß oder durch Verletzungen zerstört ist und bei der Funktion schmerzhaft wird.

Dabei erfolgt in der Regel die Resektion der zerstörten Gelenkanteile sowie die Implantation eines künstlichen Hüftgelenkes. Dieses Hüftgelenk besteht in der Regel aus Kunststoff- und Metallteilen. Bei dem Kunststoff handelt es sich in der Regel um Polyethylen, bei den Metallen um geschmiedete Stähle sowie insbesondere um Titan-Legierungen.

Bei jeder Bewegung des künstlichen Hüftgelenkes im Körper kommt es zu feinen Abriebpartikeln. Diese feinen Partikel werden an das Gewebe der Umgebung abgegeben. Der Körper bemüht sich dann, diese Mikropartikel zu neutralisieren und abzutransportieren. Dieses geschieht durch Fremdkörperriesenzellen. Der Abtransport erfolgt dann über das Lymphgefäßsystem in den übrigen Organismus.

Die Isolierung und Neutralisierung dieser Mikropartikel führt zu erheblichen Gewebeveränderungen. Es kann zu einer Osteolyse, d.h. Knochenauflösung der benachbarten Knochenabschnitte der Prothese kommen. Heute wird diese Gewebeveränderung im benachbarten Knochen durch die Abriebpartikel als wichtige Ursache für Lockerungen von Endoprothesen angesehen. Nach einem Zeitraum von 10 bis 20 Jahren erkennt man dann schwerwiegende Knochenveränderungen.

Es wurde bereits versucht, den Anfall der Mikropartikel zu vermindern, um die Standfestigkeit der Prothesen zu erhöhen.

Eine Optimierung der Gleitpaarungen in jahrzehntelanger Forschung hat eine wesentliche Verbesserung der Gelenkfunktion erbracht. So wurde beispielsweise Keramik als Gleitpartner in die Endoprothetik eingeführt. Weiterhin erfolgten insbesondere Fortschritte in der zementfreien Implantation von Hüftgelenkendoprothesen. Dabei stellt ein wesentlicher Fortschritt die Tatsache dar, daß im Bereich der Hüftpfanne ein festes Inlay aus Titan oder einer Titan-Legierung unter Verklemmung in die knöcherne Pfanne eingebracht wird. In diese künstliche Gelenkpfanne wird dann ein paßgerechter halbschalenförmiger Einsatz aus Polyethylen eingesetzt. Im Bereich des Oberschenkels erfolgt die Fixation des künstlichen Hüftgelenkes durch einen Schaft, der in den Markraum des Oberschenkelknochens eingebracht wird. Auf dem Ende des Prothesenschaftes wird ein kugelförmiger Kopf mittels einer Konuspassung fixiert.

Zusätzlich kann der Kopf noch ein halsartiges Gebilde aufweisen, das eine konische Aufnahme für einen Konus des Prothesenschaftes aufweist, wobei verschiedene Halslängen erhältlich sind. Der Kopf besteht in der Regel aus Stahl oder Keramik.

Bei der Funktion des künstlichen Hüftgelenkes kommt es zu einer Bewegung des Kopfes in der Gelenkpfanne. Untersuchungen zeigen, daß es bei jedem Schritt zu feinsten Abriebpartikeln kommen kann.

Im Laufe der Jahre kommt es dann zu einer Verdünnung der Gelenkpfanne bzw. des Einsatzes und zu großen Abriebmengen. Die Reaktion des Organismus besteht dann häufig darin, möglichst viele Gefäße und reaktionsfähiges Gewebe in dem Abriebbereich vorzuhalten, um hier Abriebpartikel durch Fremdkörperriesenzellen abzutransportieren. Dieses gelingt jedoch in der Regel nicht ausreichend. In dem neuen Gelenkbereich finden sich nicht selten nach langer Implantationszeit einerseits pastenartige amorphe Massen, die nicht nur aus Abrieb, sondern auch aus Eiweiß- und Fettbestandteilen bestehen. Andererseits kommt es zu einer Verstärkung der umgebenden Gefäße. Diese Gefäßneubildung und der Versuch, die Fremdkörperpartikel abzutransportieren, führen dann zur Auflösung von Knochenstrukturen und zur Lockerung.

Wechseloperationen führen dann wieder zu einer gewissen Festigkeit. Die erneute Lockerung geschieht jedoch schneller, als dieses nach der Erstimplantation zu beobachten ist.

Es gibt auch andere Ursachen für Lockerungen. So wurde beispielsweise der als Verankerungsmaterial dienende Knochenzement auch als Ursache für eine Lockerung identifiziert. Dieses hat dazu geführt, daß zementfreie Implantationen vermehrt durchgeführt werden. Dabei wird versucht, eine möglichst hohe primäre Stabilität zwischen den Prothesenkomponenten und dem Knochen zu erreichen. Hierfür wird eine möglichst gute Paßform angestrebt, die man herbeizuführen versucht, indem man einen möglichst guten Sitz für die zu verankernden Komponenten herbeiführt. Die Einarbeitung des Sitzes in den Knochen erfolgt zumeist von Hand. Andererseits ist es heute auch möglich, sehr genau mit Hilfe von Operationsrobotern im Schaft des Oberschenkelknochens den Prothesensitz zu gestalten.

Die klinische Erfahrung zeigt, daß auch bei den zementfreien Prothesen die Abriebproblematik greift und zu einer Lockerung der Prothesenkomponenten führen kann.

Die GB 2 315 222 A offenbart eine Hüftgelenkprothese mit einem Schaftteil und einem Kopfteil. Diese sind getrennt voneinander im Oberschenkelknochen und im Beckenknochen anbringbar. Dabei umfaßt das Kopfteil eine im einen Lagerkörper vormontierte Kugel. Die Kugel hat eine Aufnahme, in die ein Zapfen des Schaftteiles einsetzbar ist, um Schaffteil und Kopfteil miteinander zu verbinden. Abriebpartikel werden an das Gewebe in der Umgebung abgegeben.

Die US 5,514,182 A offenbart eine Hüftgelenkprothese mit einer halbdurchlässigen Membrane zum Kapseln der Gelenkflächen des Gelenks. Die halbdurchlässige Membrane ermöglicht die Zirkulation von natürlicher Körperflüssigkeit zu den Gelenkflächen des Gelenks zu Zwecken der Schmierung, wobei sie die Verteilung von Abriebspartikeln der Gelenkflächen im Körper verhindert. Zusätzlich isoliert die Membrane die Gelenkflächen von anderen Abriebspartikeln, sowie Fragmenten von Knochenzement und/oder von eingewachsenem Knochenmaterial, das mit der Grenzfläche zwischen Prothese und Knochen verbunden ist. Die Membrane trägt einen oder mehrere Stabilisierungsringe, die verhindern sollen, daß die Membrane zwischen den Gelenkflächen der Prothese eingefangen wird. Die Membrane wird nach Implantation der beiden Gelenkteile durch Verschrauben mit den Gelenkteilen verbunden. Dies ist mühselig und fehlerträchtig.

Davon ausgehend liegt der Erfindung die Aufgabe zugrunde, eine Hüftgelenkprothese mit verringerter Lockerungsneigung und erhöhter Standfestigkeit zur Verfügung zu stellen, die einfacher und sicherer implantierbar ist.

Die Aufgabe wird durch eine Hüftgelenkprothese mit den Merkmalen des Anspruches 1 gelöst. Vorteilhafte Ausgestaltungen der Hüftgelenkprothese sind in den Unteransprüchen angegeben.

Die erfindungsgemäße Hüftgelenkprothese hat
- ein femorales Teil mit einem Schaft zum Verankern im Markraum eines Oberschenkelknochens und einem Kopf am distalen Ende des Schaftes,
- ein acetabulares Teil zum Verankern im Beckenknochen mit einer Gelenkpfanne, die einen Einsatz aufweist, in dem der Kopf des femoralen Teiles gelenkig gelagert ist,
- eine Gelenkkapsel aus einem flexiblen Folien- und/oder Gewebematerial, die einenends am Einsatz der Gelenkpfanne und anderenends an der Kugel und/oder einem Hals der Kugel festgelegt ist, so daß sie eine Bewegung des Kopfes in der Gelenkpfanne zuläßt und verhindert, daß Abrieb aus der Lagerung des Kopfes in der Gelenkpfanne nach außen gelangt und
- eine vormontierte Einheit aus dem Einsatz, dem Kopf und der einenends am Einsatz und anderenends am Kopf und/oder an einem damit verbundenen Hals befestigten Gelenkkapsel, welche Einheit mit dem Einsatz in die Gelenkpfanne eingesetzt ist und mit dem Kopf und/oder dem Hals in eine Konusverbindung mit dem Schaft gebracht ist.

Erfindungsgemäß überbrückt eine künstliche Gelenkkapsel das femorale Teil und das acetabulare Teil und schottet damit die Lagerung des Kopfes in der Gelenkpfanne ab, so daß dort entstehender Abrieb aus der Hüftgelenkprothese nicht austreten kann. Hierfür kann die Gelenkkapsel beispielsweise abdichtend mit einem Einsatz der Gelenkpfanne und mit dem Kopf und/oder einem den Kopf mit dem Schaft verbindenden Hals verbunden sein. Dabei muß die Gelenkkapsel eine Struktur und/oder ein Material haben, das eine ausreichende Beweglichkeit des - vorzugsweise im wesentlichen kugelförmigen - Kopfes in der Gelenkpfanne gestattet. Zum anderen muß das Material und die Befestigung der Gelenkkapsel so dicht sein, daß Mikropartikel nicht hindurchwandern. Diese Mikropartikel können eine Größe von wenigen µm oder darunter aufweisen.

Für die Gelenkkapsel kommt insbesondere ein Folien- und/oder Gewebematerial in Betracht. Dabei kann es sich um einen Kunststoff und/oder ein Metall und/oder ein natürliches Material handeln. Insbesondere kommen PTFE-Fasern und/oder PETP-Fasern in Betracht. Besonders vorteilhaft dürfte die Verwendung von Goretex® (ein PTFE-Material; Bezugsquelle: Gore) oder Dacron® (ein PETP-Material; Bezugsquelle: DuPont) sein (®: eingetragene Marke). Goretex® hat sich in den letzten Jahrzehnten bereits als Material für Gefäßprothesen bewährt. Es handelt sich um ein Fasermaterial bzw. Gewebe, das unterschiedliche Porengrößen aufweisen kann. Weiterhin besteht die Möglichkeit, innen- und/oder außenseitig eine Beschichtung durchzuführen, die zu einer weitgehenden Abdichtung von Poren führt. Ferner haben sich auch Dacron®-Gefäßprothesen bewährt (ebenfalls ein Faser- bzw. Gewebematerial). Dacron® kann als Material für die Gelenkkapsel ebenfalls innen und/oder außen beschichtet sein. Gegebenenfalls kann die Gelenkkapsel aus einem in vitro gezüchteten Material mit der Beschaffenheit des natürlichen Gelenkkapselgewebes bestehen.

Um möglichst viel Bewegungsspielraum zu ermöglichen, kann die Gelenkkapsel als Faltenbalg ausgeführt sein. Sie kann beispielsweise eine Mehrzahl ziehharmonikaartiger Falten aufweisen. Um Materialbrüchen im Bereich der Falten vorzubeugen, können diese Verstärkungen aufweisen.

Weiter kann die Gelenkkapsel eine von der Lagerung des Kopfes in der Gelenkpfanne weggerichtete Ausbuchtung aufweisen. Die Ausbuchtung befindet sich bei implantierter Hüftgelenkprothese unterhalb der Lagerung. Sie bildet ein Speichervolumen, das Abriebpartikel aufnehmen kann. Weiterhin kann hier vorteilhaft eine körperverträgliche Substanz vorhanden sein, die diese Abriebpartikel festhält und ein Zurückweichen in das Gelenk damit verhindert (Fliegenfängerfunktion).

Ferner besteht die Möglichkeit, die Gelenkkapsel in der Weise auszugestalten, daß sie einen schlauchartigen Abgang hat. Vorzugsweise weist sein Ende einen öffenbaren Verschluß auf, beispielsweise eine Pfropf. Über diesen Pfropf kann dann die Hüftgelenkprothese durch Punktion und Vorschieben eines Katheters erreicht und gegebenenfalls ausgespült und gereinigt werden.

Insbesondere bei einer vollständigen Abkapselung kann durch körperverträgliche Substanzen innerhalb der Gelenkkapsel eine Art Schmierung erfolgen, um so den Abrieb der Gelenkkomponenten möglichst klein zu halten.

Die sichere Befestigung der Gelenkkapsel am acetabularen Teil, insbesondere an einem Einsatz der Gelenkpfanne, kann in einer umlaufenden Nut erfolgen. Die Gelenkkapsel kann in dieser Nut mit einem Wulst und/oder mit einem Sicherungsring festgelegt sein. In gleicher Weise kann die Befestigung der Gelenkkapsel am femoralen Teil, beispielsweise am Kopf und/oder am Hals erfolgen. Auch dieser kann eine umlaufende Nut aufweisen, in der die Gelenkkapsel zu liegen kommt, gegebenenfalls mit einem Wulst und/oder mit einem Sicherungsring. Um ein Herausziehen der künstlichen Gelenkkapsel aus dieser Befestigung zu verhindern, kann ein über die Befestigung hinausstehender Rand der Gelenkkapsel einen Wulst aufweisen, der verhindert, daß das Material unter dem Sicherungsring herausgezogen werden kann.

Eine ganz besonders vorteilhafte Ausgestaltung der Erfindung hat eine vormontierte Einheit aus einem Einsatz, einem Kopf und einer einenends am Einsatz und anderenends am Kopf und/oder Hals befestigten Kapsel mit einem Aufnahmekonus im Hals. Diese vormontierte Einheit bildet das eigentliche Gelenkteil, das im Bereich des Hüftgelenkes als letzte Komponente eingebracht wird. Nach Implantation der Gelenkpfanne im Bereich des Beckens sowie des Schaftes im Markraum des Oberschenkelknochens erfolgt dann das Einsetzen des abgekapselten Gelenkteiles. Dabei wird der Einsatz mit der daran hängenden Gelenkkapsel in die Gelenkpfanne eingesetzt, um anschließend den Kopf mit der daran hängenden Kapsel auf den Konus des Schaftes zu setzen. Um diesen Vorgang leichter zu gestalten, weist der Hals gemäß einer Ausgestaltung einen seitlichen Schlitz auf, durch den der Konus eingesetzt wird. Darauf kann der Konus tiefer in die Aufnahme eingeschoben und damit gesichert werden.

Gemäß einer Ausgestaltung ist die Kugel einschnappend oder nicht einschnappend in den Einsatz eingesetzt.

Gemäß einer Ausgestaltung ist der Einsatz in die Gelenkpfanne unter Ausbildung eines Paßsitzes oder einer Schnappverbindung eingesetzt.

Durch besondere Ausbildung der Außenfläche der Gelenkkapsel kann das Anwachsverhalten des Gewebes optimiert werden. Es ist davon auszugehen, daß es zur Ausbildung von Schleimbeuteln kommt, die eine verstärkte Beweglichkeit des künstlichen Hüftgelenkes gegenüber dem umgebenden Gewebe ermöglichen. Durch eine besonders rauhe Oberfläche, z. B. faserartige Oberfläche, kann ein Anwachsen des Gewebes gefördert werden.

Weiterhin kann die Hüftgelenkprothese mindestens einen elektronischen Sensor integriert haben, um die Funktion der Hüftgelenkprothese zu überwachen. Die Meßdaten können mittels einer Telemetriervorrichtung, die in der Hüftgelenkprothese integriert oder gesondert implantiert werden kann, aus dem menschlichen Körper heraus übertragen werden. Zu den zu überprüfenden Funktionen des Gelenkes gehört beispielsweise die Intaktheit der Gelenkkapsel, die Erwärmung des Gelenkes, gehören pathologische Bewegungen von femoralem Teil und acetabularem Teil im Knochen u.a. Hierdurch kann der Patient vor möglichem Versagen oder Überbelastungen der Hüftgelenkprothese gewarnt werden.

Die Erfindung wird nachfolgend anhand der anliegenden Zeichnungen bevorzugter Ausführungsformen näher erläutert. In den Zeichnungen zeigen:
- Fig. 1: eine Hüftgelenkprothese in einen Patienten implantiert in teilweisem Längsschnitt;
- Fig. 2: eine zweite Ausführung der Hüftgelenkprothese in teilweisem Längsschnitt;
- Fig. 3: eine dritte Ausführung der Hüftgelenkprothese in teilweisem Längsschnitt;
- Fig. 4: Gelenkkapsel derselben Hüftgelenkprothese in einem vergrößerten Schnitt.

Bei der nachfolgenden Erläuterung verschiedener Ausführungen sind übereinstimmende Elemente mit denselben Bezugsziffern versehen. Die zugehörige Beschreibung hat für sämtliche Ausführungen Gültigkeit, die dieselbe Bezugsziffer aufweisen.

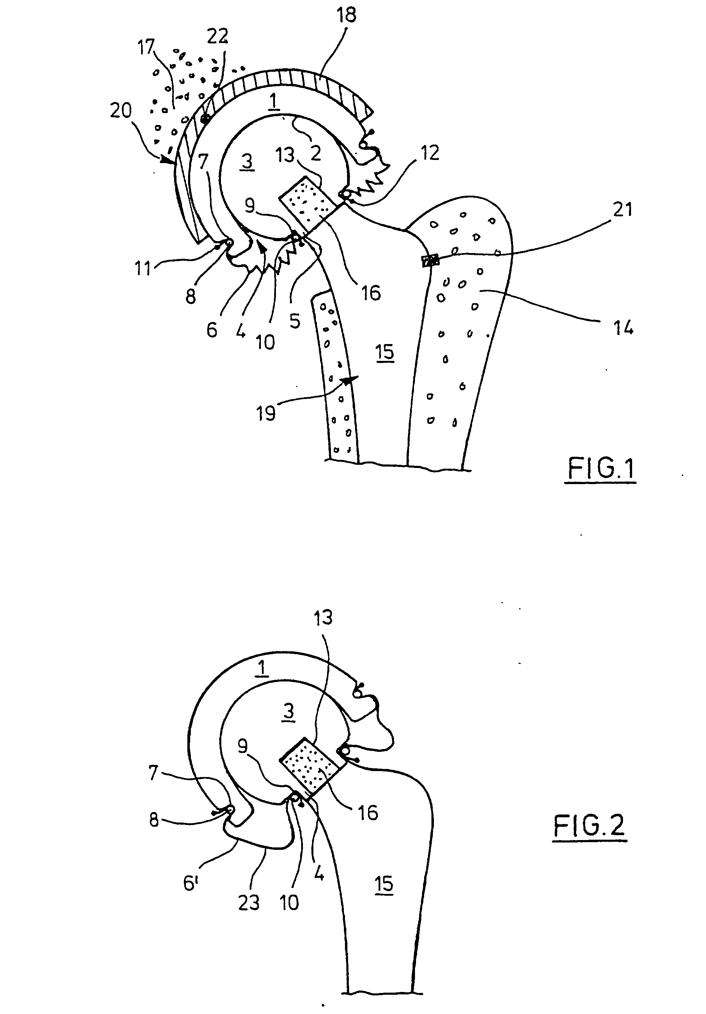

Gemäß Fig. 1 hat die Hüftgelenkprothese einen schalenförmigen Einsatz 1, der beispielsweise aus Polyethylen hergestellt sein kann. Der Einsatz 1 hat innen eine kugelschalenförmige Lagerfläche 2. Darin ist ein kugelförmiger Kopf 3 gelagert. Dieser hat an einem aus einer Öffnung 4 des Einsatzes 1 herausragenden Abschnitt einen vorstehenden, zylindrischen Hals 5.

Eine Gelenkkapsel 6 in Form eines Faltenbalges ist einenends in einer umlaufenden Nut 7 an der Außenseite des Einsatzes 1 in der Nähe der Öffnung 4 mittels eines Sicherungsringes 8 festgelegt. Anderenends ist die Gelenkkapsel 6 in einer umlaufenden Nut 9 im Übergangsbereich von Kopf 3 und Hals 5 mittels eines weiteren Sicherungsringes 10 festgelegt. An den Enden hat die Gelenkkapsel wulstförmige Verdickungen 11, 12, die ein Herausziehen aus den Nuten 7, 9 verhindern sollen.

Kugel 3 und Hals 4 haben eine zum Hals konzentrische, leicht konische Aufnahme 13.

Der Einsatz 1, die Kugel 3 und die Gelenkkapsel 6 bilden eine vormontierte Einheit. Gegebenenfalls kann die Kugel 3 durch Einschnappen im Einsatz 1 gesichert sein.

In einen Oberschenkelknochen 14 ist ein Schaft 15 der Hüftgelenkprothese implantiert. Dieser hat distal einen vorstehenden Konus 16. Dieser ist in die konische Aufnahme 13 gesteckt. Aufnahme 13 und Konus 16 bilden eine den Sitz der Kugel 3 auf dem Schaft 15 sichernde Konuspassung.

In einen Beckenknochen 17 ist eine Gelenkpfanne 18 implantiert, die insbesondere aus Titan oder einem anderen metallischen Material bestehen kann. Die Gelenkpfanne 18 kann mittels - nicht gezeigter - Knochenschrauben im Hüftknochen 17 fixiert sein. In die Gelenkpfanne 18 ist der Einsatz 1 eingesetzt, vorzugsweise unter Ausbildung eines Paßsitzes oder einer Schnappverbindung.

Kugel 3, Hals 4 und Schaft 15 werden insgesamt auch als femorales Teil 19 und Gelenkpfanne 18 und Einsatz 1 werden insgesamt auch als acetabulares Teil 20 der Hüftgelenkprothese bezeichnet.

Die Anbringung im Hüftgelenksbereich erfolgt vorzugsweise so, daß zunächst der Schaft 15 und die Gelenkpfanne 18 implantiert werden und daß dann das Gelenkteil mit den Komponenten 1, 3 und 6 auf den Konus 16 aufgesteckt bzw. in die Pfanne 18 eingesetzt wird.

Im Gebrauch entsteht Abrieb durch Bewegung der Kugel 3 im Einsatz 1. Dieser wird von der Gelenkkapsel 6 im Inneren der Hüftgelenkprothese 1 zurückgehalten, wodurch eine Lockerung der Prothese vermieden und die Standfestigkeit derselben erhöht wird. Zur Überwachung der Prothesenfunktion können Sensoren vorhanden sein, beispielsweise ein Sensor 21 zwischen Schaft 15 und Knochen 14 oder ein Sensor 22 zwischen Gelenkpfanne 18 und Beckenknochen 17. Diese Sensoren 21, 22 dienen insbesondere der Messung der Kraftübertragung, wobei die Meßdaten Rückschlüsse auf Überlastungen oder Lockerungen zulassen.

Gemäß Fig. 2 hat die Hüftgelenkprothese eine Kapsel 6' mit einer vom Einsatz 1 und dem Kopf 3 wegweisenden, umlaufenden Ausbuchtung 23. Diese begünstigt zum einen die Beweglichkeit des Kopfes 3 im Einsatz 1 und bildet zum anderen ein Speichervolumen für Abrieb.

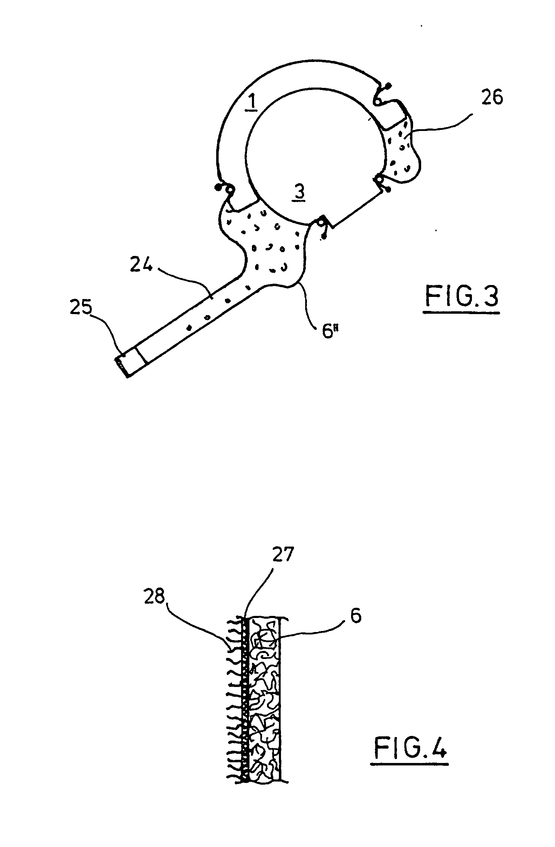

Gemäß Fig. 3 hat eine Hüftgelenkprothese eine Gelenkkapsel 5'' mit einem schlauchförmigen, seitlichen Abgang 24, der einen pfropfenförmigen Verschluß 25 aufweist. Die Gelenkkapsel 5'' ist mit einem biologisch kompatiblen Schmiermittel 26 für die Prothese gefüllt. Eine Spülung bzw. ein Austausch ist über den Pfropfen 26 möglich.

Gemäß Fig. 4 hat die Gelenkkapsel 6 eine Wand aus PTFE-Material (Goretex u.ä.). Dieses ist beispielsweise auf der Außenseite mit einer Beschichtung 27 versehen, die das Gewebematerial undurchlässig für Partikel machen soll. Außerdem hat das Material außen eine Aufrauhung 28, die das Anwachsen von Gewebematerial fördern soll.

## Patentansprüche

1. Künstliches Hüftgelenk mit
- einem femoralen Teil (19) mit einem Schaft (15) zum Verankern im Markraum eines Oberschenkelknochens (14) und einem kugelförmigen Kopf (3) am distalen Ende des Schaftes (15),
- einem acetabularen Teil (20) zum Verankern im Beckenknochen (17) mit einer Gelenkpfanne (18), die den Kopf (3) des femoralen Teiles (19) gelenkig lagert, und
- einer Gelenkkapsel (6) aus einem flexiblen Folien- und/oder Gewebematerial, die einenends an der Gelenkpfanne (18) und anderenends an dem Kopf (3) und/oder einem Hals (4) des Kopfes (3) festgelegt ist, so daß sie eine Bewegung des Kopfes (3) in der Gelenkpfanne (18) zuläßt und verhindert, daß Abrieb aus der Lagerung des Kopfes (3) in der Gelenkpfanne (18) nach außen gelangt, **dadurch gekennzeichnet, daß**
- eine vormontierte Einheit aus dem Einsatz (1), dem Kopf (3) und der einenends am Einsatz (1) und anderenends am Kopf (3) und/oder an einem damit verbundenen Hals (4) befestigten Gelenkkapsel (6) mit dem Einsatz (1) in die Gelenkpfanne (18) eingesetzt ist und mit dem Kopf (3) und/oder dem Hals (4) in eine Konusverbindung mit dem Schaft (15) gebracht ist.

2. Hüftgelenkprothese nach Anspruch 1, bei der die Gelenkkapsel (6) ein Faltenbalg ist.

3. Hüftgelenkprothese nach Anspruch 2, bei der die Gelenkkapsel (6) eine Verstärkung an mindestens einer Falte aufweist.

4. Hüftgelenkprothese nach einem der Ansprüche 1 bis 3, bei der Hals (4) einen seitlichen Schlitz aufweist, durch den ein Konus (16) des femoralen Teils (19) in eine konische Aufnahme (13) des Halses (4) eingesetzt und darauf tiefer in die Aufnahme (13) eingeschoben ist.

5. Hüftgelenkprothese nach einem der Ansprüche 1 bis 4, bei der die Kugel (3) einschnappend oder nicht einschnappend in den Einsatz (1) eingesetzt ist.

6. Hüftgelenkprothese nach einem der Ansprüche 1 bis 5, bei der der Einsatz (1) in die Gelenkpfanne (18) unter Ausbildung eines Paßsitzes oder einer Schnappverbindung eingesetzt ist.

7. Hüftgelenkprothese nach einem der Ansprüche 1 bis 6, bei dem die Verbindung zwischen der Gelenkkapsel (6) und dem acetabularen Teil (20) und/oder dem femoralen Teil (19) einen Wulst (11, 12) und/oder eine Nut (7, 9) und/oder einen Sicherungsring (8, 10) aufweist.

8. Hüftgelenkprothese nach einem der Ansprüche 1 bis 7, bei der die Gelenkkapsel (6") einen schlauchartigen Abgang (24) aufweist.

9. Hüftgelenkprothese nach einem der Ansprüche 1 bis 8, bei der die Gelenkkapsel (6") und/oder der Abgang (24) einen öffenbaren Verschluß (25) aufweist, durch den ein Zugang zum Innenraum der Gelenkkapsel (6") möglich ist.

10. Hüftgelenkprothese nach einem der Ansprüche 1 bis 9, bei der die Gelenkkapsel (6') und/oder der Abgang (24) ein Speichervolumen (23) aufweist.

11. Hüftgelenkprothese nach Anspruch 10, bei der das Speichervolumen in einer Ausbuchtung (23) der Kapsel (6') vorhanden ist.

12. Hüftgelenkprothese nach Anspruch 10 oder 11, bei der das Speichervolumen (23) eine Abriebpartikel festhaltende Substanz enthält.

13. Hüftgelenkprothese nach einem der Ansprüche 1 bis 12, bei der die Gelenkkapsel (6) aus einem Kunststoff und/oder aus einem Metall und/oder aus einem natürlichen Material besteht.

14. Hüftgelenkprothese nach einem der Ansprüche 1 bis 13, bei der die Gelenkkapsel (6) aus PTFE-Fasern und/oder PETP-Fasern besteht.

15. Hüftgelenkprothese nach einem der Ansprüche 1 bis 14, bei der die Gelenkkapsel (6) aus Goretex® und/oder Dacron® besteht.

16. Hüftgelenkprothese nach einem der Ansprüche 1 bis 15, bei der die Gelenkkapsel (6) aus einem teilweise durchlässigen Material besteht, das undurchlässig für Abrieb aus der Lagerung des Kopfes (3) in der Gelenkpfanne (18) ist.

17. Hüftgelenkprothese nach Anspruch 16, bei der die Gelenkkapsel (6) eine Beschichtung (27) zum Abdichten von Poren und/oder Festhalten von Abrieb aufweist.

18. Hüftgelenkprothese nach Anspruch 17, bei der die Gelenkkapsel (6) außen eine das Anwachsen von Gewebe förderndes Material und/oder Oberflächenbeschaffenheit und/oder Beschichtung (28) aufweist.

19. Hüftgelenkprothese nach einem der Ansprüche 1 bis 18, bei der die Gelenkkapsel (6) eine Schmiersubstanz enthält.

20. Hüftgelenkprothese nach einem der Ansprüche 1 bis 19, bei der mindestens ein elektronischer Sensor (21, 22) zum Ermitteln einer Gelenkfunktion vorhanden ist.

21. Hüftgelenkprothese nach Anspruch 20, bei der der Sensor (21, 22) mit einer Telemetrievorrichtung zum Übermitteln von Meßdaten an die Umgebung des menschlichen Körpers verbunden ist.

## Claims

1. An artificial hip joint assembly comprising:
- a femoral component (19) with a stem (15) for being anchored in the medullary space of a femur (14) and a spherical head (3) on the distal end of the stem (15),
- an acetabular component (20) for being anchored in the pelvic bone (17) with an acetabular cup (18) which has an insert in which the head (3) of the femoral component (19) is pivotedly supported,
- an articular capsule (6) made of a flexible foil and/or tissue material, which is located at the acetabular cup at one end and at the head (3) and/or a neck (4) of the head (3) at the other end so as to allow the head (3) to move in the acetabular cup (18) and to prevent wear debris from the bearing zone of the head (3) in the acetabular cup (18) from migrating to the outside, **characterized in that**
- a pre-assembled unit comprising the insert (1), the head (3), and the articular capsule (6) attached to the insert (1) at one end and to the head and/or the neck (4) connected thereto, at the other end, has its insert (1) placed in the acetabular cup (18) and has its head (3) and/or its neck (4) brought into a cone connection to the stem (15).

2. The prosthetic hip joint assembly according to claim 1 wherein the acetabular cup (6) is a corrugated bellows.

3. The prosthetic hip joint assembly according to claim 2 wherein the acetabular cup (6) has a reinforcement on at least one corrugation.

4. The prosthetic hip joint assembly according to any of claims 1 to 3, wherein the head (4) has a lateral slot through which the cone (16) of the femoral component (19) is inserted into a conical seat (13) of the neck (4) and then pushed deeper into the seat (13).

5. The prosthetic hip joint assembly according to any of claims 1 to 4, wherein the ball (3) is placed in the insert (1) by snapping it in or without snapping it in.

6. The prosthetic hip joint assembly according to any of claims 1 to 5, wherein the insert (1) is placed in the acetabular cup (18) by forming a snug fit or a snap connection.

7. The prosthetic hip joint assembly according to any of claims 1 to 6, wherein the connection between the articular capsule (6) and the acetabular component (20) and/or the femoral component (19) has a bead (11, 12) and/or a groove (7, 9) and/or a locking collar (8, 10).

8. The prosthetic hip joint assembly according to any of claims 1 to 7, wherein the articular capsule (6") has a hose-like extension (24).

9. The prosthetic hip joint assembly according to any of claims 1 to 8, wherein the articular capsule (6") and/or the extension (24) has a closure (25) adapted to be opened, through which an access is possible to the interior of the articular capsule (6").

10. The prosthetic hip joint assembly according to any of claims 1 to 9, wherein the articular capsule (6') and/or the extension (24) has a storage volume (23).

11. The prosthetic hip joint assembly according to claim 10, wherein the storage volume exists in a bulged-out portion (23) of the capsule (6').

12. The prosthetic hip joint assembly according to claim 10 or 11, wherein the storage volume (23) contains a wear particle retaining substance.

13. The prosthetic hip joint assembly according to any of claims 1 to 12, wherein the articular capsule (6) is made of a plastic and/or a metallic and/or a natural material.

14. The prosthetic hip joint assembly according to any of claims 1 to 13, wherein the articular capsule (6) is made of PTFE fibres and/or PETP fibres.

15. The prosthetic hip joint assembly according to any of claims 1 to 14, wherein the articular capsule (6) is made of Goretex® and/or Dacron®.

16. The prosthetic hip joint assembly according to any of claims 1 to 15, wherein the articular capsule (6) is made of a partially permeable material which is impermeable to wear debris from the bearing zone of the head (3) in the acetabular cup (18).

17. The prosthetic hip joint assembly according to claim 16, wherein the articular capsule (6) has applied to it a coat (27) for sealing its pores and/or retaining wear debris.

18. The prosthetic hip joint assembly according to claim 17, wherein the articular capsule (6) has applied to it a material and/or surface finish and/or coat (28) which promotes the bonding of tissue thereto.

19. The prosthetic hip joint assembly according to any of claims 1 to 18, wherein the articular capsule (6) contains a lubricating substance.

20. The prosthetic hip joint assembly according to any of claims 1 to 19, wherein there is at least one electric sensor (21, 22) for determining a joint function.

21. The prosthetic hip joint assembly according to claim 20, wherein the sensor (21, 22) is connected to a telemetering device for the transfer of data measured to the surroundings of the human body.

## Revendications

1. Articulation artificielle de la hanche avec
- une partie fémorale (19) avec une tige (15) pour l'ancrage dans le canal médullaire d'un fémur (14), et une tête (3) sphérique sur l'extrémité distale de la tige (15),
- une partie acétabulaire (20) pour l'ancrage dans l'os de la hanche (17) avec un acétabulum (18) qui présente un insert (1) dans lequel la tête (3) de la partie fémorale (19) repose de façon articulée,
- une capsule articulaire (6) en matériau en feuille et/ou en tissu flexible, qui est fixée à une extrémité sur l'acétabulum (18) et à l'autre extrémité sur la tête (3) et/ou une gorge (4) de la tête (3) de sorte qu'elle autorise et empêche un mouvement de la tête (3) dans l'acétabulum (18), de sorte que les particules abrasées en provenance de l'appui de la tête (3) dans l'acétabulum (18) sont dirigées vers l'extérieur, **caractérisée en ce que**
- une unité prémontée à partir de l'insert (1), de la tête (3) et de la capsule articulaire (6) fixée à une extrémité sur l'insert (1) et à l'autre extrémité sur la tête (3) et/ou sur une gorge (4) qui lui est liée, laquelle unité avec l'insert (1) est mise en place dans l'acétabulum (18) et est, avec la tête (3) et/ou la gorge (4), amenée dans une liaison conique avec la tige (15).

2. Prothèse de la hanche selon la revendication 1, dans laquelle la capsule articulaire (6) est un soufflet à plis.

3. Prothèse de la hanche selon la revendication 2, dans laquelle la capsule articulaire (6) présente un renfort sur au moins un pli.

4. Prothèse de la hanche selon une des revendications 1 à 3, dans laquelle la gorge (4) présente une fente latérale à travers laquelle un cône (16) de la partie fémorale (19) est inséré dans un logement conique (13) de la gorge (4) et puis est inséré plus profondément dans le logement (13).

5. Prothèse de la hanche selon une des revendications 1 à 4, dans laquelle la bille (3) est mise en place dans l'insert (1) par encliquetage ou sans encliquetage.

6. Prothèse de la hanche selon une des revendications 1 à 5, dans laquelle l'insert (1) est mis en place dans l'acétabulum (18) avec formation d'un ajustement serré ou d'un assemblage à encliquetage.

7. Prothèse de la hanche selon une des revendications 1 à 6, dans laquelle le raccordement entre la capsule articulaire (6) et la partie acétabulaire (20) et/ou la partie fémorale (19) présente un bourrelet (11, 12) et/ou une rainure (7, 9) et/ou une bague de blocage (8, 10).

8. Prothèse de la hanche selon une des revendications 1 à 7, dans laquelle la capsule articulaire (6") présente une sortie (24) de type tuyau.

9. Prothèse de la hanche selon une des revendications 1 à 8, dans laquelle la capsule articulaire (6") et/ou la sortie (24) présente une obturation (25) pouvant être ouverte, à travers laquelle un accès à l'espace intérieur de la capsule articulaire (6") est possible.

10. Prothèse de la hanche selon une des revendications 1 à 9, dans laquelle la capsule articulaire (6') et/ou la sortie (24) présente un volume de stockage (23).

11. Prothèse de la hanche selon la revendication 10, dans laquelle le volume de stockage est présent dans une échancrure (23) de la capsule (6').

12. Prothèse de la hanche selon la revendication 10 ou 11, dans laquelle le volume de stockage (23) contient une substance retenant les particules d'abrasion.

13. Prothèse de la hanche selon une des revendications 1 à 12, dans laquelle la capsule articulaire (6) est constituée d'une matière plastique et/ou d'un métal et/ou d'un matériau naturel.

14. Prothèse de la hanche selon une des revendications 1 à 13, dans laquelle la capsule articulaire (6) est constituée de fibres PTFE et/ou de fibres PETP.

15. Prothèse de la hanche selon une des revendications 1 à 14, dans laquelle la capsule articulaire (6) est constituée de Goretex ® et/ou de Dacron ®.

16. Prothèse de la hanche selon une des revendications 1 à 15, dans laquelle la capsule articulaire (6) est constituée d'un matériau partiellement perméable qui est imperméable aux particules abrasées en provenance de l'appui de la tête (3) dans l'acétabulum (18).

17. Prothèse de la hanche selon la revendication 16, dans laquelle la capsule articulaire (6) présente un revêtement (27) pour boucher les pores et/ou retenir les particules abrasées.

18. Prothèse de la hanche selon la revendication 17, dans laquelle la capsule articulaire (6) présente à l'extérieur un matériau et/ou un état de surface et/ou un revêtement (28) favorisant la croissance de tissus.

19. Prothèse de la hanche selon une des revendications 1 à 18, dans laquelle la capsule articulaire (6) contient une substance lubrifiante.

20. Prothèse de la hanche selon une des revendications 1 à 19, dans laquelle il y a un capteur électronique (21, 22) pour l'obtention d'une fonction d'articulation.

21. Prothèse de la hanche selon la revendication 20, dans laquelle le capteur (21, 22) est raccordé à un équipement de télémétrie pour la transmission de données de mesure à l'environnement du corps humain.
